# EUROPEAN PATENT SPECIFICATION

(11) **EP 0 413 544 B1**
(45) Date of publication and mention of the grant of the patent: **02.01.1997**
(21) Application number: 90308898.7
(22) Date of filing: 13.08.1990
(51) Int. Cl.: C07D 473/32, C07D 473/18, A61K 31/52, C07D 239/50, C07D 239/20

(54) **Purine derivatives, process for their preparation, pharmaceutical compositions and intermediates**
Purinverbindungen, Verfahren zu ihrer Herstellung, pharmazeutische Präparate und Zwischenverbindungen
Dérivés de purine, procédé pour leur préparation, compositions pharmaceutiques et intermédiaires

(30) Priority: 17.08.1989 GB 8918827
(43) Date of publication of application: 20.02.1991
(73) Proprietor: BEECHAM GROUP PLC, Brentford, Middlesex TW8 9EP (GB)
(72) Inventor: Harnden, Michael Raymond, Storrington, West Sussex RH20 3HY (GB); Wyatt, Paul Graham, Cheam Village, Surrey (GB); Jennings, Leslie John Arthur, SmithKline Beecham, Epsom, Surrey KT18 5XQ (GB)
(74) Representative: Tocher, Pauline

(56) References cited:
- EP-A- 0 242 482
- EP-A- 0 294 069
- EP-A- 0 298 601
- EP-A- 0 313 289
- CHEMICAL ABSTRACTS, vol. 109, no. 15, 10th October 1988, page 749, abstract no. 129567p, Columbus, Ohio, US; M.R. HARNDEN et al.: "Synthesis of 9-(3-hydroxypropoxy)guanine, a novel antiviral acyclonuclleoside" & TETRAHEDRON LETT. 1988, 29(6), 701-4

## Description

The present invention relates to novel compounds which are of potential use as antiviral agents, to a process for their preparation and to their use as pharmaceuticals.

EP-A-242482 (Beecham Group p.l.c.), the subject matter of which is incorporated herein by reference, discloses antiviral compounds of formula (A) and pharmaceutically acceptable salts thereof: 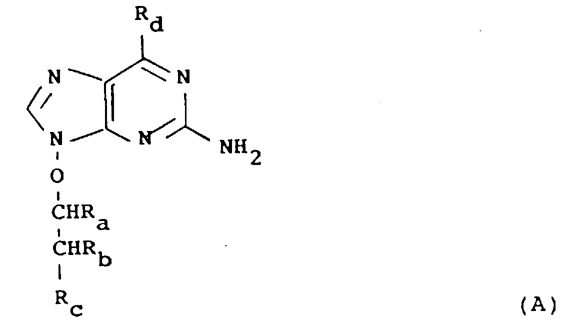 wherein
- Rₐ: is hydrogen or CH₂OH;
- R_{b}: is hydrogen or, (when R₁ is hydrogen), hydroxy or CH₂OH;
- R_{c}: is CH₂OH or, (when R₁ and R₂ are both hydrogen), CH(OH)CH₂OH;
- R_{d}: is hydrogen, hydroxy, amino or ORₑ
wherein
- Rₑ: is C₁₋₆ alkyl, phenyl or phenyl C₁₋₂ alkyl either of which phenyl moieties may be substituted by one or two halo, C₁₋₄ alkyl or C₁₋₄ alkoxy groups;
and in which any OH groups in Rₐ, R_{b} and/or R_{c} may be in the form of O-acyl, phosphate, cyclic acetal or cyclic carbonate derivatives thereof.

Compounds wherein R_{d} is other than hydroxy are pro-drugs for the compounds of formula (A) wherein R_{d} is hydroxy. Example 1 describes the compound of formula (I) wherein Rₐ and R_{b} are both hydrogen, R_{c} is CH₂OH and R_{d} is hydroxy which is 9-(3-hydroxyprop-1-oxy)guanine, hereinafter referred to as E1.

It has now been discovered that certain derivatives of E1 are pro-drugs for E1, having improved gastrointestinal absorption properties.

Accordingly, the present invention provides a compound of formula (I) or a pharmaceutically acceptable salt thereof: 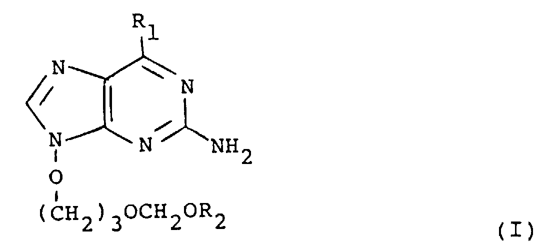 wherein
- R₁: is hydrogen or hydroxy; and
- R₂: is C₁₋₆ alkyl.

Suitable values for R₂ alkyl groups include methyl, ethyl, n- and iso-propyl, n-, iso-, sec- and tert-butyl and pentyl (all possible isomers), particularly iso-propyl.

Pharmaceutically acceptable salts are as described in EP-A-242482.

The compounds of formula (I) including their alkali metal salts may form solvates such as hydrates and these are included wherever a compound of formula (I) or a salt thereof is herein referred to.

It will be appreciated that, when R₁ is hydroxy in formula (I) the compound exists in the predominant tautomeric form of structure (IA):

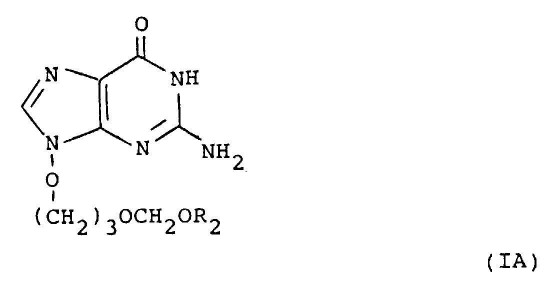

The invention also provides a process for the preparation of a compound of formula (I), or a pharmaceutically acceptable salt thereof, which process comprises either
i) imidazole ring closure of a compound of formula (II): 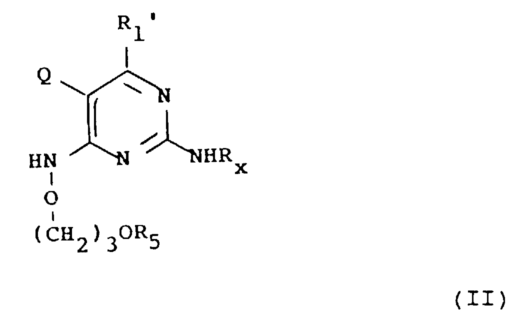 wherein Q is a group capable of cyclising to form an imidazole ring, such as amino or an amino derivative, for example, formylamino; or
ii) pyrimidine ring closure of a compound of formula (III): 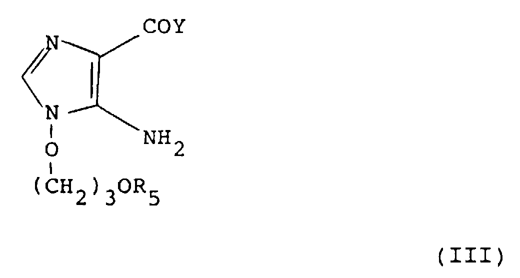 wherein Y is amino or C₁₋₆ alkoxy, with a condensing agent capable of cyclising to form a pyrimidine ring having a 2-NHRₓ substituent, resulting in a compound of formula (I) wherein R₁ is hydroxy; or
iii) condensing a compound of formula (IV): 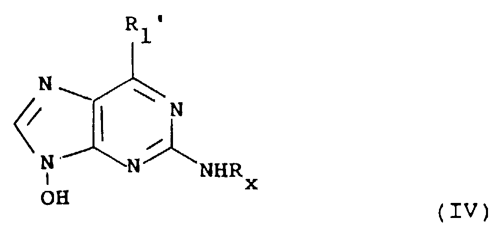 with a side chain intermediate of formula (V):

   R₅O(CH₂)₃Z (V)

   wherein Z is a leaving group;
   and wherein, in formulae (II) to (V), R₅ is CH₂OR₂ or a group or atom convertible thereto, R₁′ is R₁ or a group or atom convertible thereto, Rₓ is hydrogen or an amino protecting group; and thereafter, when desired or necessary, converting R₅ and/or R₁′, when other than CH₂OR₂ and/or R₁, to CH₂OR₂ and/or R₁ respectively, and/or converting R₅ and/or R₁ when CH₂OR₂ and/or R₁ to other CH₂OR₂ and/or R₁ and/or converting Rₓ when an amino protecting group, to hydrogen.

Process i) may be carried out, preferably when Q is formylamino, using a cyclisation condensing agent, such as diethoxymethyl acetate or triethyl orthoformate, or by fusion.

Process ii) is preferably carried out in accordance with the methods described in EP-A-242482, the subject matter of which is incorporated herein by reference.

Process iii) may be carried out with suitable values for Z including hydroxy and halo, such as chloro, bromo and iodo, preferably iodo; or other groups readily displaceable by nucleophiles, such as mesyloxy or tosyloxy. The reaction preferably takes place in an inert solvent, such as dimethylformamide at 0-50°C, preferably ambient temperature. When Z is hydroxy, the reaction takes place in the presence of a dehydrating agent, such as diethyl azodicarboxylate in the presence of triphenylphosphine. When Z is halo, the reaction preferably takes place in the presence of a base, such as potassium carbonate.

Examples of conversions of variable groups are as follows:

### R₁′-R₁

a) An R₁ hydroxy group may be converted to R₁′ is chloro, by chlorination using a reagent such as phosphorus oxychloride, preferably in the presence of tetraethylammonium chloride and dimethylaniline (as acid acceptor) in CH₃CN at reflux temperatures, according to the method described by M.J. Robins and B. Ozanski, Can. J. Chem, 59, 2601 (1981).
b) When R₅ is other than CH₂OR₂, an R₁′ chloro group may be converted to R₁ is hydroxy by hydrolysis using aqueous mineral acid, such as hydrochloric acid, or more preferably, using an organic acid, such as formic acid at elevated temperature, suitably 70-150°C, preferably around 100°C.
c) An R₁′ chloro group may be converted to R₁′ is methoxy by reaction with sodium methoxide. The R₁′ is methoxy group may in turn be converted to R₁′ is hydroxy by hydrolysis using non-acidic methods, preferably using mercaptoethanol.
d) An R₁′ alkoxy group, such as methoxy, may also be converted to R₁ hydroxy by the methods of D.R. Haines, J. Med. Chem. 1987, 30, 943 and K.K. Ogilvie and H.R. Hanna, Can. J. Chem. 1984, 62, 2702.

### R₅-CH₂OR₂

a) R₅ hydrogen may be converted to CH₂OR₂ by conventional procedures for preparing acetals, using LCH₂OR₂ wherein L is a leaving group, such as chloro or acetate.
b) R₅ hydrogen may be replaced by a protecting group, which may be removed by conventional deprotection methods, and then subsequently converted to CH₂OR₂ as in a) above.

Suitable examples of protecting groups and processes for their removal, are as described in EP-A-242482. Particularly suitable protecting groups include the benzyl group, removed by catalytic hydrogenation; the acetate group removed by acid hydrolysis, 2M HCl in ethanol; or the t-butyldimethylsilyl group removable by 80% acetic acid at elevated temperature, around 90°C.

### Rₓ′-Rₓ

Rₓ′ may be formyl, which may be converted to Rₓ is hydrogen by hydrolysis, preferably under basic conditions.

It will be appreciated that the above conversions may take place in any desired or necessary order, having regard to the final desired compound of formula (I), and that interconversions involving acid conditions could affect the OCH₂OR₂ moiety in formula (I).

It is normally preferred that R₅ in the aforedescribed processes is CH₂OR₂ as defined.

Intermediates of formula (II) may be prepared from a corresponding compound of formula (VI): 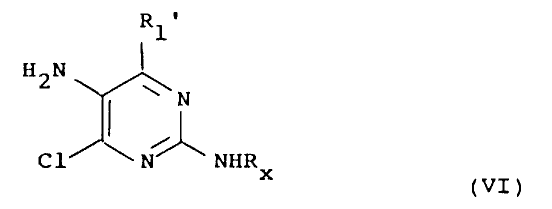 and via intermediates of formula (V) wherein Z is OH, as hereinbefore defined, according to the methods described in EP-A-242482 i.e. by converting the compound of formula (V) wherein Z is OH to the phthalimidooxy derivative followed by reaction with methylhydrazine, as described in the Descriptions hereinafter.

The compound of formula (VI) wherein R₁′ is chloro and Rₓ is hydrogen, is a known compound as described by Temple et. al., J. Org. Chem., 40 (21), 3141, 1975.

Intermediates of formula (III) may be prepared according to the methods described in EP-A-242482.

Compounds of the formula (IV) are prepared as described in EP-A-313289 (Beecham Group p.l.c.) from compounds of formula (VI) wherein the 5-amino group is formylated, by reaction with R₆ONH₂ wherein R₆ is a protecting group, to give a compound of formula (VII): 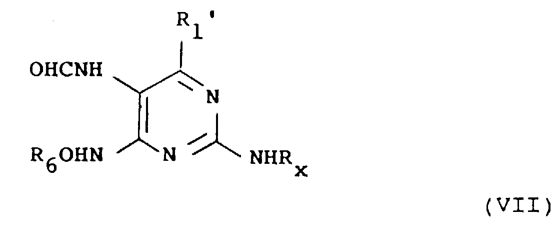 which may be cyclised with diethoxymethyl acetate, to give a compound of formula (IV) wherein the OH group is protected. Suitable values for R₆ include benzyl, removable by hydrogenation, and the tetrahydropyran-2-yl group removable by treatment with 80% acetic acid, at ambient temperature.

Intermediates of the formula (V) wherein Z is hydroxy are known compounds or are prepared by analogous methods to those used for structurally similar known compounds.

Intermediates of formulae (II), (III) and (V) but wherein Z is replaced by an aminooxy group, and wherein R₅ is CH₂OR₂, are believed to be novel and form an aspect of the invention.

Pharmaceutically acceptable salts may be prepared in conventional manner, for example, in the case of acid addition salts, by reaction with the appropriate organic or inorganic acid.

The compounds of the invention are of potential use in the treatment of infections caused by viruses, especially herpesviruses such as herpes simplex type 1, herpes simplex type 2, varicella-zoster and Epstein-Barr virus.

Compounds of the invention may be formulated for use in a pharmaceutical composition. Accordingly, in a further aspect of the invention, there is provided a pharmaceutical composition which comprises a compound of formula (I) or pharmaceutically acceptable salt thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that may be administered in a single dose. A suitable dosage unit might contain from 50 mg to 1 g of active ingredient, for example 100 to 500 mg.

Such doses may be administered 1 to 4 times a day or more usually 2 or 3 times a day. The effective dose of compound will in general be in the range of from 1.0 to 20 mg/kg of body weight per day or more usually 2.0 to 10 mg/kg per day.

No unacceptable toxicological effects are indicated at the above described dosage levels.

The invention also provides a method of treating viral infections in a human or non-human animal, which comprises administering to the animal an effective, non-toxic amount of a compound of formula (I) or a pharmaceutically acceptable salt thereof.

The invention also provides a compound of formula (I) or a pharmaceutically acceptable salt thereof for use as an active therapeutic substance, in particular for the treatment of viral infections.

The compounds of the invention are also believed to exhibit a synergistic antiviral effect in conjunction with interferons; and combination products comprising these two components for sequential or concomitant administration, by the same or different routes, are therefore within the ambit of the present invention.

The following examples illustrate the invention; the following descriptions illustrate the preparation of intermediates.

### Description 1 (Intermediates for Example 1)

### a) 3-(Isopropoxymethoxy)propanol

A solution of 1,3-propanediol (19.4g, 255mmol) in dry tetrahydrofuran (100ml) under nitrogen at 0-5°C was treated with sodium hydride (2.04g, 85mmol) and stirred for 1 hour. Chloromethylisopropyl ether (9g, 83mmol) in dry tetrahydrofuran (15ml) was added over 15 min. Stirring was continued for a further 2 hours. The reaction mixture was filtered and the filtrate was evaporated in vacuo. The residue was purified by column chromatography on silica eluting with dichloromethane: methanol (97:4) to give the title compound as a colourless oil. (5.3g, 44%). νₘₐₓ (Film) 3400, 2910, 2860, 2805, 2760, 1460, 1440, 1425, 1410 and 1380cm⁻¹; δ_{H} (CDCl₃) 1.17 (3H, s, CH₃), 1.20 (3H, s,CH₃), 1.84 (2H, quintet, J=6Hz CH₂CH₂CH₂), 2.3 (1H, br s, D₂O exchangeable OH), 3.72 (2H, t, J=6Hz, CH₂OCH₂OCH(CH₃)₂) 3.76 (2H, t, J=6Hz,CH₂ON), 3.86 (1H, m, CH(CH₃)₂), 4.70 (2H, s, OCH₂O).

### b) N-[3-(Isopropoxymethoxy)propoxy]phthalimide

A mixture of [3-(isopropoxymethoxy)propanol (5.3g, 35.8mmol), N-hydroxyphthalimide (5.83g, 35.8mmol) and triphenylphosphine (10.48g, 40mm0l) in dry tetrahydrofuran (75ml) at 0-5°C; was treated with a solution of diethyl azodicarboxylate (6.96g, 40mmol) in dry tetrahydrofuran (15ml) over 15min. The reaction mixture was stirred at ambient temperature for 18hr. The solvent was removed in vacuo and the residue was dissolved in diethyl ether (100ml) and cooled to 5°C for 2hr. The solid was filtered off, the filtrate evaporated in vacuo, and the residue purified by column chromatography on silica eluting with hexane: ethyl acetate (70:30) to give the title compound as an oil (9.3g, 88%). νₘₐₓ (Film) 2980, 2940, 2900, 1740, 1475 and 1380 cm⁻¹; δ_{H} (CDCl₃) 1.17 (3H, s, CH₃), 1.19 (3H, s, CH₃), 2.06 (2H, quintet, J=6.3Hz CH₂CH₂CH₂), 3.77 (2H, t, J=6.3Hz, CH₂OCH₂OCH(CH₃)₂), 3.88 (1H, septet, J=6.3Hz, OCH(CH₃)₂), 4.32 (2H, t, J=6.3Hz CH₂ON), 4.73 (2H, s, OCH₂O), 7.78 (4H, m, ArH). (Found: C, 16.53; H, 6.59; N, 4.71%: C₁₅H₁₉NO₅ requires: C, 16.42; H, 6.52; N, 4.77%).

### c) N-[3-(Isopropoxymethoxy)propoxyamine

A solution of N-[3-(isopropoxymethoxy)propoxy]phthalimide (9.3g, 31.7mmol) in dry dichloromethane (70ml) at ambient temperature was treated with N-methylhydrazine (2.2g, 47.8mmol) and stirred for 2 hours. The reaction mixture was filtered, the filtrate evaporated in vacuo and the residue purified by column chromatography on silica eluting with hexane: ethyl acetate (60:40) to give the title compound as a colourless oil (4.2g, 82%). νₘₐₓ (Film) 3310, 3220, 3160, 2960, 2920, 2870, 1580, 1465 and 1380 cm⁻¹; δ_{H} (CDCl₃) 1.10 (3H, s, CH₃), 1.18 (3H, s, CH₃), 1.80 (2H, quintet, J=6.3Hz CH₂CH₂CH₂), 3.52 (2H, t, J=6.3Hz, CH₂OCH₂OCH(CH₃)₂), 3.66 (2H, t, J=6.3Hz, CH₂ON), 3.8 (1H, septet, J=6.3Hz OCH(CH₃)₂), 4.65, (2H, s, OCH₂O), 5.2-55 (2H, br s, D₂O exchangeable NH₂).

### d) 4-Chloro-2,5-diformamido-6-[(3-isopropoxymethoxy)propoxy]aminopyrimidine

A mixture of 4,6-dichloro-2,5-diformamidopyrimidine (2.9g, 12.3mmol), 3-(isopropoxymethoxy)propoxyamine (2.0g, 12.2mmol) and N,N-diisopropylethylamine (4.3ml, 3.19g, 24.6mmol) in diglyme (50ml) was heated to 100° for 3hr. The cooled reaction mixture was filtered, and the filtrate was evaporated in vacuo. The residue was purified by column chromatography on silica eluting with ethyl acetate:hexane (1:1) then ethyl acetate to give the title compound as a yellow gum, which crystallised from ethyl acetate (3.0g, 75%) mp 132.4°C. νₘₐₓ(KBr) 3250, 2960, 2910, 2870, 1705, 1645, 1590, 1565, 1495, 1460 and 1380cm⁻¹; δH [(CD₃)₂SO], 1.08 (3H, s, CH₃), 1.10 (3H, s, CH₃), 1.84 (2H, quintet, J=6.3 Hz, CH₂CH₂CH₂), 3.58 (2H, t, J 6.3 Hz, CH₂OCH₂OCH(CH₃)₂), 3.76 (1H, septet, J=6.3 Hz, OCH(CH₃)₂), 3.93 (2H, t, J 6.3 Hz, CH₂ON), 4.61 (2H, s, OCH₂O), 8.14 (1H, s, NHCHO), 9.25 (1H, br.s, NHCHO), 9.39 (1H, br.s, D₂O exchangeable NHOCH₂), 10.6-11.0 (2H, br, D₂O exchangeable, 2 x NHCHO). (Found: C, 43.20; H, 5.55; N, 19.24%. C₁₃H₂₀N₅O₅Cl requires: C, 43.15; H, 5.57; N, 19.35%).

### Description 2 (Intermediates for Example 2)

### a) 3-(Methoxymethoxy)propanol

A solution of 1,3-propanediol (25.1g, 330mmol) in dry tetrahydrofuran (10ml) was treated with sodium hydride (80%, 3.3g, 110mmol) at room temperature under nitrogen. The solution was stirred at ambient temperature for 30min. then chloromethylmethyl ether (8.85g, 110mmol) was added dropwise with stirring in dry tetrahydrofuran (15ml), maintaining the temperature at 10°C (ice bath). The reaction was stirred for 2hr. then filtered and the filtrate was evaporated in vacuo. The residue was purified by column chromatography on silica eluting with dichloromethane: methanol (97:3) to give the title compound as a colourless oil (7.0g, 53%). νₘₐₓ(Film) 3400, 2920, 2880, 2760, 1460, 1435, and 1380cm⁻¹; δ_{H}(CDCl₃) 1.8 (2H, quintet, J=6Hz, CH₂CH₂CH₂), 3.1 (1H, s, D₂O exchangeable, OH), 3.35 (3H, s, OCH₃), 3.7 (4H, m, CH₂OH, CH₂OCH₂OCH₃), 4.65 (2H, s, OCH₂O).

### b) N-[3-(Methoxymethoxy)propoxy]phthalimide

A solution of 3-(methoxymethoxy)propanol (5g, 41.6mmol), triphenylphosphine (13.1g, 50mmol) and N-hydroxyphthalimide (7.25g, 44mmol) in dry tetrahydrofuran (150ml) was cooled to 0-5°C and treated with diethyl azodicarboxylate (8.7g, 50mmol) in dry tetrahydrofuran (10ml) over ½hr. The reaction was then stirred at ambient temperature for 18hr. The solvent was removed in vacuo and the residue was dissolved in diethyl ether (100ml) and the mixture cooled to 0-5°C for 3hr. The solid was removed by filtration and the filtrate was evaporated in vacuo. The residue was purified by column chromatography on silica eluting with hexane: ethyl acetate (70:30) to give the title compound as an oil (4.5g, 41%). νₘₐₓ(Film) 2960, 2900, 1800, 1740, 1620, 1480 and 1380cm⁻¹; δ_{H}(CDCl₃) 2.07 (2H, quintet, J=6.3 Hz, CH₂CH₂CH₂), 3.38 (3H, s, OCH₃), 3.77 (2H, t, J=6.3 Hz, CH₂OCH₂OCH₃), 4.33 (2H, t, J=6.3 Hz, CH₂ON), 4.66 (2H, s, OCH₂O), 7.83 (4H, m, ArH).

### c) 3-Methoxymethoxypropoxyamine

A solution of N[3-(methoxymethoxy)propoxy]phthalimide (4.5g, 17mmol) in dry dichloromethane (50ml) was cooled to 0-5° and treated with N-methylhydrazine (1.2g, 26mmol). The reaction mixture was stirred for 2hr then filtered and the filtrate was evaporated in vacuo. The residue was purified by column chromatography on silica eluting with hexane:ethyl acetate (60:40) to give the title compound as a colourless oil (1.1g, 50%). νₘₐₓ (Film) 3310, 3240, 3160, 2960, 2910, 1590, 1465, and 1380cm⁻¹; δ_{H}(CDCl₃) 2.07 (2H, quintet, J=6.3Hz, CH₂CH₂CH₂), 3.38 (3H, s, CH₃O), 3.50 (2H, t, J=6.3Hz, CH₂OCH₂OCH₃), 3.70 (2H, t, J 6.3 Hz, CH₂ONH₂). 4.66 (2H, s, OCH₂O), 5.2-5.5 (2H, br.s, D₂O exchangeable, NH₂).

### d) 4-Chloro-6-[3-(methoxymethoxy)propoxy]amino-2,5-diformamidopyrimidine

A mixture of 4,6-dichloro-2,5-diformamidopyrimidine (1.75g, 7.4mmol), 3-(methoxymethoxy)propoxyamine (1.0g, 7.4mmol) and N,N-diisopropylethylamine (2g, 15.5mmol) in dry diglyme was heated to 100°C for 2hr. The cooled reaction mixture was filtered and the filtrate was evaporated in vacuo. The residue was purified by column chromatography on silica eluting with ethyl acetate:hexane (50:50) then ethyl acetate to give the title compound as a yellow oil which was crystallised from ethyl acetate (1.2g, 50%) mp 136-8°C. νₘₐₓ(KBr) 3240, 2920, 2880, 1705, 1640, 1590, 1565, 1495, 14650, 1410, and 1380cm⁻¹; δ_{H} [(CD₃)₂SO] 1.85 (2H, quintet, J=6.3Hz, CH₂CH₂CH₂), 3.24 (3H, s, OCH₃), 3.58 (2H, t, J=6.3Hz, CH₂OCH₂OCH₃), 3.94 (2H, t, J=6.3Hz, CH₂ONH), 4.5 (2H, s, OCH₂O), 8.15 (1H, s, NHCHO), 9.26 (1H, d, J=9.6Hz, collapses to singlet on D₂O, NHCHO), 9.4 (1H, s, D₂O exchangeable NHOCH₂), 10.73 (1H, s, D₂O, exchangeable NHCHO), 10.84 (1H, d, J=9.6Hz, D₂O exchangeable, NHCHO).

### Description 3 (Intermediate for Examples 4 and 5)

### 3-(Ethoxymethoxy)propan-1-ol

A solution of chloroethylethyl ether (50mmol) in dry tetrahydrofuran (10ml) was added dropwise at 0-5°C to a stirred solution of 1,3-propanediol (150mmol) in dry tetrahydrofuran (100ml) and N,N-diisopropylethylamine (75mmol). The mixture was stirred for 2 hours at room temperature, the solvent was evaporated in vacuo and the residue was purified by column chromatography on silica, eluting with dichloromethane:methanol (98:2) to give the title compound as a colourless oil (5.6g, 83%); νₘₐₓ(Film) 3420, 2980, 2940, 2880, 1480, 1445, 1390 and 1180 cm⁻¹; δ_{H}(CDCl₃) 1.20 (3H, t, J=6.5Hz, CH₃CH₂), 1.8 (2H, m, CH₂CH₂CH₂), 3.2 (1H, s, OH), 3.6 (6H, m, CH₂OH, CH₂O and CH₃CH₂), 4.8 (2H, s, OCH₂O).

### Examples

The following compounds of formula (I) were prepared.

| Example No | R₁ | R₂ |
|---|---|---|
| 1 | H | (CH₃)₂CH |
| 2 | H | CH₃ |
| 3 | OH | (CH₃)₂CH |
| 4 | OH | C₂H₅ |
| 5 | H | C₂H₅ |

### Example 1

### 2-Amino-9-[3-(isopropoxymethoxy)propoxy)purine

a) A mixture of 4-chloro-2,5-diformamido-6-[3-(isopropoxymethoxy)propoxyamino]pyrimidine (3.3g, 9.12mmol) and diethoxymethyl acetate (10ml) was heated at 120°C for 1hr. The solution was then evaporated and the residue taken up into methanol (30ml) and concentrated aqueous ammonia (3ml). After 1hr. at 20°C the solvent was removed and the residue purified by column chromatography on silica gel eluting with hexane:ethyl acetate (1:1) to afford 6-chloro-2-formamido-9-[3-(isopropoxymethoxy)propoxy]purine (1.89g; 60%), νₘₐₓ (KBr) 2970, 1697, 1609, 1581, 1503, 1383cm⁻¹; δ_{H} [(CD₃)₂SO], 1.10 (6H, d, J=6.3Hz, 2 x CH₃), 1.97 (2H, quintet, J=6.3Hz, CH₂CH₂CH₂), 3.67 (2H, t, J=6.3Hz, CH₂OC), 3.77 (1H, septet, J=6.3Hz, CH), 4.49 (2H, t, J=6.3Hz, CH₂ON), 4.65 (2H, s, OCH₂O), 8.81 (1H, s, H-8), 9.37 (1H, d, J=9.1Hz, CHO), 11.30 (1H, br.s, J=9.1Hz, NH).
b) A mixture of 6-chloro-2-formamido-9-[3-(isopropoxymethoxy)propoxy]purine (1.86g, 5.40mmol), 10% palladium on charcoal (190mg), triethylamine (3.8ml, 27mmol) and methanol (30ml) was stirred under an atmosphere of hydrogen at 20°C for 4hr. The suspension was filtered, the catalyst washed with chloroform and the combined filtrates evaporated under reduced pressure. The residue was dissolved in dichloromethane (50ml), washed with saturated brine-water (2:1), dried (magnesium sulphate) and evaporated. The residue was dissolved in 0.5M sodium methoxide in methanol (21.6ml) and heated at reflux for 1hr. The solution was taken to neutrality using AMBERLITE IR 120(H) and evaporated. The residue was purified by column chromatography on silica gel eluting with chloroform-methanol (20:1) affording the title compound (630mg, 41%) after recrystallisation from acetone-hexane; νₘₐₓ(KBr) 3333, 3192, 1661, 1623, 1577, 1516, 1433 cm⁻¹; δ_{H} [(CD₃)₂SO] 1.09 (6H, d,J 6.05 Hz, 2 x CH₃), 1.95 (2H, quintet, J 6.6 and 6.3 Hz, CH₂CH₂CH₂), 3.63 (2H, t, J=6.3Hz, CH₂OC), 3.77 (1H, septet, J=6.0Hz, CH), 4.39 (2H, J=6.6Hz, CH₂ON), 4.64 (2H, s, OCH₂O), 6.69 (2H, br.s, D₂O exchangeable, NH₂), 8.31 (1H, s, H-8), 8.59 (1H, s, H-6). Found: C, 51.21; H, 6.84; N, 24.82%; C₁₂H₁₉N₅O₃ requires: C, 51.22; H, 6.82; N, 24.89%.

### Example 2

### 2-Amino-9-[3-(methoxymethoxy)propoxyl]purine

a) A mixture of 4-chloro-2,5-diformamido-6-[3-(methoxymethoxy)propoxy]pyrimidine (1.2g, 3.80mmol) and diethoxymethyl acetate (10ml) was heated at 120°C for 1hr, then cooled and evaporated. The residue was taken up into methanol (10ml) and concentrated aqueous ammonia (1ml) and stirred at 20°C for 1hr. The solvent was then removed in vacuo. The residue was purified by column chromatography on silica gel eluting with ethyl acetate-hexane (1:1) affording 6-chloro-2-formamido-9-[3-(methoxymethoxy)propoxy]purine (0.45g, 1.42mmol) which was dissolved in methanol (30ml), and treated with excess triethylamine (1ml). The flask was flushed with nitrogen, palladium on carbon (10%, 40mg) was added and the mixture was hydrogenated at S.T.P. until hydrogen uptake ceased. The reaction mixture was filtered, the filtrate evaporated in vacuo and the residue purified by crystallisation from methanol to give 2-formamido-9-[3-(methoxymethoxy)propoxy]purine as colourless crystals (0.4g, 100%) mp. 115-116°C. λₘₐₓ (MeOH) 231 (ε 23,880), 255 (8120) and 290nm (9430); νₘₐₓ(KBr) 3120, 3070, 2930, 2810, 1690, 1610, 1510, 1445, 1410, and 1375cm⁻¹. δ_{H}[(CD₃)₂SO] 1.98 (2H, quintet, J=6.3Hz, CH₂CH₂CH₂), 3.25 (3H, s, OCH₃), 3.67 (2H, t, J=6.3Hz, CH₂OCH₂OCH₃), 4.5 (2H, t, J 6.3 Hz, CH₂ON), 4.58 (2H, s, OCH₂O), 8.73 (1H, s, 8-H), 8.98 (1H, s, 6-H), 9.42 (1H, d, J=9.6Hz, collapses to singlet on D₂O NHCHO), 11.1 (1H, d, J=9.6Hz, D₂O exchangeable NHCHO). (Found: C, 47.11; H, 5.27; N, 24.18%, C₁₁H₁₅N₅O₄ requires: C, 46.97; H, 5.37; N, 24.90%).
b) A solution of 2-formamido-9-[3-(methoxymethoxy)propoxy]purine (0.4g, 1.42mmol) in methanol (15ml) was treated with sodium methoxide solution (0.5M, 5ml) and heated to reflux for 2hr. The cooled reaction mixture was neutralised with AMBERLITE IR 120H resin, filtered and the solvent was removed in vacuo.

The residue was dissolved in ethyl acetate (50ml), washed with water (2 x 20ml), dried (MgSO₄), filtered and the filtrate evaporated in vacuo. The residue was purified by column chromatography on silica eluting with ethyl acetate, followed by crystallisation from acetone/hexane to give the title compound as colourless crystals (0.2g, 55%) mp 80-1°C. λₘₐₓ (MeOH) 223 (ε 26,375), 245 (5020) and 310 nm (7320); νₘₐₓ(KBr) 3320, 3180, 3080, 2940, 2880, 1645, 1610, 1580, 1505, 1465, and 1430cm⁻¹; δ_{H} [(CD₃)₂SO] 1.95 (2H, quintet, J=6.3Hz, CH₂CH₂CH₂), 3.25 (3H, s, OCH₃), 3.63 (2H, t, J=6.3Hz, CH₂OCH₂OCH₃), 4.40 (2H, t, J=6.3Hz, CH₂ON), 4.57 (2H, s, OCH₂O), 6.68 (2H, br.s, D₂O exchangeable NH₂), 8.3 (1H, s, 8-H), 8.59 (1H, s, 6-H). (Found: C, 47.34, H 6.08% C₁₀H₁₅N₅O₃ requires: C, 47.42; H, 59.7%).

### Example 3

### 9-[3-(Isopropoxymethoxy)propoxy]guanine

a) Diethyl azodicarboxylate (1.24ml, 7.87mmol) was added to a cooled solution of 2-[(bis-t-butoxycarbonyl)amino]-9-hydroxy-6-methoxypurine (2.0g, 5.23mmol), 3-(isopropoxymethoxy)propanol (0.85g, 5.75mmol), and triphenylphosphine (2.06g, 7.87mmol) in THF (50ml). The reaction mixture was stirred for 16 hours, and then evaporated under reduced pressure. The residue was purified by column chromatography on silica gel (hexane-acetone, 3:1) affording 2-[(bis-t-butoxycarbonyl)amino]-9-[3-(isopropoxymethoxy)propoxy]-6-methoxypurine (2.23g, 83%). νₘₐₓ (KBr), 2975, 1788, 1761, 1597 and 1389 cm⁻¹; δ_{H}[(CD₃)₂SO] 1.90 (6H, d, J=6.3Hz, 2xCH₃), 1.40 (18H, s, 6xCH₃), 1.96 (2H, quintet, J=6.3Hz, OCH₂CH₂CH₂O), 3.64 (2H, t, J=6.3Hz, CH₂O), 3.78 (1H, quintet, J=6.3Hz, CHO), 4.08 (2H, s, OCH₃), 4.48 (2H, t, J=6.3Hz, CH₂ON), 4.63 (2H, s, OCH₂O), 8.74 (1H, s, H-8). (Found: C, 54.09; H, 7.59; N, 13.65%. C₂₃H₃₇N₅O₈ requires: C, 53.99; H, 7.30; N, 13.69%).
b) A solution of 2-[(bis-t-butoxycarbonyl)amino]-9-[3-(isopropoxymethoxy)propoxy]-6-methoxypurine in 0.5M sodium methoxide in methanol (33ml) was heated at reflux temperature for 16 hours. 2-Mercaptoethanol (1.3ml) was then added and the reaction mixture heated for a further 48 hours. The suspension was cooled, neutralised and evaporated to dryness. The residue was purified by reverse phase chromatography eluting with water then water-methanol-concentrated aqueous ammonia (10:1:1), to afford the title compound (390mg; 34%) after recrystallisation from water. νₘₐₓ(KBr) 3332, 3131, 3168, 1695, 1599, 1586 and 1384 cm⁻¹; δ_{H}[(CD₃)₂SO] 1.09 (6H, d, J=6.1Hz, 2xCH₃), 1.91 (2H, quintet, J=6.6Hz, 6.3Hz, OCH₂CH₂CH₂O), 3.61 (2H, t, J=6.3Hz, CH₂O), 3.77 (1H, septet, J=6.0Hz, CHO), 4.32 (2H, t, J=6.6Hz, CH₂ON), 4.63 (2H, s, OCH₂O), 6.58 (2H, br.s, NH₂), 7.93 (1H, s, H-8), 0.64 (1H, br.s, H-1). (Found: C, 48.58; H, 6.39; N, 23.44%. C₁₂H₁₉N₅O₄ requires C: 48.47; H, 6.45; N, 23.56%).

### Example 4

### 9-[3-(Ethoxymethoxy)propoxy]guanine

a) A mixture of 2-[(bis-t-butoxycarbonyl)amino]-9-hydroxy-6-methoxypurine (2.85g, 7.48mmol), 3-(ethoxymethoxy)propan-1-ol (1.0g, 7.46mmol) and triphenylphosphine (2.62g, 10mmol) in dry tetrahydrofuran (50ml) was cooled to 0-5°C and treated with a solution of diethyl azodicarboxylate (1.74g, 10mmol) in dry tetrahydrofuran (10ml), the solution was then allowed to stand at room temperature overnight. The solvent was then removed in vacuo and the residue was purified by column chromatography on silica, eluting with hexane:acetone (95:5) to give 2-[(bis-t-butoxycarbonyl)amino]-9-[3-(ethoxymethoxy)propoxy]-6-methoxypurine as a yellow gum (1.7g, 46%). νₘₐₓ(Film) 3120, 2980, 2940, 2880, 1795, 1760, 1595, 1475, 1410, 1390, 1370, 1280 and 1255 cm⁻¹; δ_{H}[(CD₃)₂SO] 1.10 (3H, t, J=6.8Hz, CH₃CH₂), 1.4 (18H, s, CH₃x6), 1.96 (2H, m, J=6.3Hz, CH₂CH₂CH₂), 3.47 (2H, q, J=6.8Hz, CH₃CH₂), 3.64 (2H, t, J=6.3Hz, CH₂CH₂ON), 4.07 (3H, s, OCH₃), 4.47 (2H, t, J=6.3Hz, OCH₂CH₂), 4.6 (2H, s, OCH₂O), 8.74 (1H, s, 8-H). (Found: C, 52.57; H, 7.18; N, 13.92%; C₂₂H₃₅N₅O₈ requires: C, 53.10; H, 7.09; N, 14.07%) MS (70eV) m/z = 498 (MH⁺).
b) A solution of 2-[(bis-t-butoxycarbonyl)amino]-9-[3-(ethoxymethoxy)propoxy]-6-methoxypurine (0.7g, 1.4mmol) in sodium methoxide (0.5M, 25ml) was heated to reflux for 18hr. 2-Mercaptoethanol (1ml) was then added to the solution and heating was continued for a further 18hr. The cooled reaction mixture was neutralised with hydrochloric acid solution (0.5M) and the solvent was removed in vacuo. The residue was purified by column chromatography on silica eluting with chloroform:methanol (90:10) followed by crystallisation from methanol to give the title compound as colourless crystals (0.1g, 25%) mp 242-5°C. λₘₐₓ(MeOH) 250nm (14080); νₘₐₓ(KBr) 3320, 3160, 2870, 2730, 1690, 1645, 1590, 1535, 1470, 1385 and 1320cm⁻¹; δ_{H}[(CD₃)₂SO] 1.11 (3H, t, J=7.1Hz, CH₂CH₃), 1.92 (2H, m, J=6.5Hz, CH₂CH₂CH₂), 3.5 (2H, q, J=7.1Hz, CH₂CH₃), 3.61 (2H, t, J=6.3Hz, CH₂CH₂ON), 4.32 (2H, t, J=6.3Hz, OCH₂CH₂), 4.60 (2H, s, OCH₂O), 6.57 (2H, br.s, D₂O exchangeable, NH₂), 7.92 (1H, s, 8-H), 10.63 (1H, br.s, D₂O exchangeable, NH). (Found: C, 46.64; H, 5.78; N, 24.55%; C₁₁H₁₇N₅O₄ requires: C, 46.63; H, 6.05; N, 24.72%) MS (70eV) m/z = 284 (MH⁺).

### Example 5

### 2-Amino-9-[3-(ethoxymethoxy)propoxy]purine

a) A solution of 2-[(bis-t-butoxycarbonyl)amino]-6-chloro-9-hydroxypurine (2.4g, 7.4mmol), 3-(ethoxymethoxy)propan-1-ol (1.0g, 7.4mmol) and triphenylphosphine (2.62g, 10mmol) in dry tetrahydrofuran (35ml) was cooled to 0-5°C and treated with a solution of diethyl azodicarboxylate (1.75g, 10mmol) in dry tetrahydrofuran (10ml). The solution was allowed to stand at room temperature overnight, the solvent was evaporated in vacuo and the residue purified by column chromatography on silica eluting with chloroform:methanol (98:2) followed by a further column eluting with hexane:acetone (90:10) to give 2-[(bis-t-butoxycarbonyl)amino]-6-chloro-9-[3-(ethoxymethoxy)propoxy]purine as a colourless gum (1.7g, 46%). νₘₐₓ(Film) 3100, 2980, 2930, 2880, 1795, 1760, 1600, 1560, 1425, 1370, 1280 and 1155cm⁻¹; δ_{H}[(CD₃)₂SO] 1.11 (3H, t, J=7.1Hz, CH₃CH₂), 1.4 (18H, s, CH₃x6), 2.01 (2H, m, J=6.3Hz, CH₂CH₂CH₂), 3.5 (2H, q, J=7.1Hz, CH₃CH₂), 3.64 (2H, t, J=6.3Hz, CH₂CH₂ON), 4.55 (2H, t, J=6.3Hz, OCH₂CH₂), 4.6 (2H, s, OCH₂O), 9.08 (1H, s, 8-H). (Found: C, 50.28; H, 6.55; N, 13.70%; C₂₁H₃₂N₅O₇Cl requires: C, 50.24; H, 6.42; N, 13.95%); MS (70eV) m/z = 502 (MH⁺).
b) To a solution of 2-[(bis-t-butoxycarbonyl)amino]-6-chloro-9-[3-(ethoxymethoxy)propoxy]purine (1.5g, 2.98mmol) in methanol (25ml) and triethylamine (1ml) under a nitrogen atmosphere was added palladium on carbon (5%, 0.1g) and the mixture was hydrogenated at S.T.P. until hydrogen uptake ceased. The reaction mixture was filtered and evaporated in vacuo and, the residue was purified by column chromatography on silica, eluting with ethyl acetate:hexane (50:50) to give 2-[(bis-t-butoxycarbonyl)amino]-9-[3-(ethoxymethoxy)propoxy]purine as a colourless gum. (1.2g, 86%). νₘₐₓ(Film) 3100, 2980, 2930, 2880, 1790, 1735, 1600, 1575, 1480, 1455, 1390, 1370, 1280 and 1250cm⁻¹; δ_{H}[(CD₃)₂SO] 1.11 (3H, t, J=7.1Hz, CH₂CH₃), 1.38 (18H, s, CH₃x6), 1.98 (2H, m, J=6.3Hz, CH₂CH₂CH₂), 3.5 (2H, q, J=7.1Hz, CH₂CH₃), 3.65 (2H, t, J=6.3Hz, CH₂CH₂ON), 4.52 (2H, t, J=6.3Hz, OCH₂CH₂), 4.60 (2H, s, OCH₂O), 8.98 (1H, s, 8-H), 9.21 (1H, s, 6-H). (Found: C, 53.88; H, 7.26; N, 15.13%; C₂₁H₃₃N₅O₇ requires: C, 53.94, H, 7.11; N, 14.98%) MS (70eV) m/z = 468 (MH⁺).
c) 2-[(Bis-t-butoxycarbonyl)amino]-9-[3-(ethoxymethoxy)propoxy]purine (0.5g, 1.07mmol) was dissolved in sodium methoxide solution (0.5M, 30ml) and heated to reflux for 18hr. The cooled reaction mixture was evaporated in vacuo and the residue was purified by column chromatography on silica, eluting with ethyl acetate:hexane (60:40) followed by crystallisation from acetone:hexane to give the title compound as colourless crystals (0.25g, 89%) mp 73-4°C. λₘₐₓ(MeOH) 224, 246 and 310nm. (5610, 3750 and 20,700); νₘₐₓ(KBr) 3370, 3330, 3200, 3080, 2930, 2880, 1645, 1615, 1570, 1505, 1475, 1425, 1320, 1280 and 1220cm⁻¹; δ_{H}[(CD₃)₂SO] 1.11 (3H, t, J=7.1Hz, CH₂CH₃), 1.95 (2H, m, J=6.3Hz, CH₂CH₂CH₂), 3.50 (2H, q, J=7.1Hz, CH₂CH₃), 3.63 (2H, t, J=6.3Hz, CH₂CH₂ON), 4.4 (2H, t, J=6.6Hz, OCH₂CH₂), 4.61 (2H, s, OCH₂O), 6.69 (2H, br.s, D₂O exchangeable, NH₂), 8.31 (1H, s, 8-H), 8.59 (1H, s, 6-H). (Found: C, 49.48; H, 6.50; N, 26.33%; C₁₁H₁₇N₅O₃ requires: C, 49.42; H, 6.41; N, 26.20%); MS (70eV) m/z = 268 (MH⁺).

### Biological Evaluation

### Procedure

Compounds were administered as single doses of 0.2mmole/kg in 0.lml of 1% carboxymethyl cellulose by oral gavage to female Balb/c mice weighing 20g. Food was withheld from the mice for 18 hours prior to the start of the experiment. Blood was collected by cardiac puncture using heparinised syringes 15, 60 and 180 mins after dosing. Equal volumes (0.2ml) from 3 mice were pooled at each time point and 0.6ml of ice-cold ethanol was added. Following chilling at -20°C and centrifugation, 0.5ml of supernatant was dried under reduced pressure. The sample was then reconstituted with 0.5ml of 0.4M NH₄OAc (pH 6.0) and analysed by HPLC.

### Results

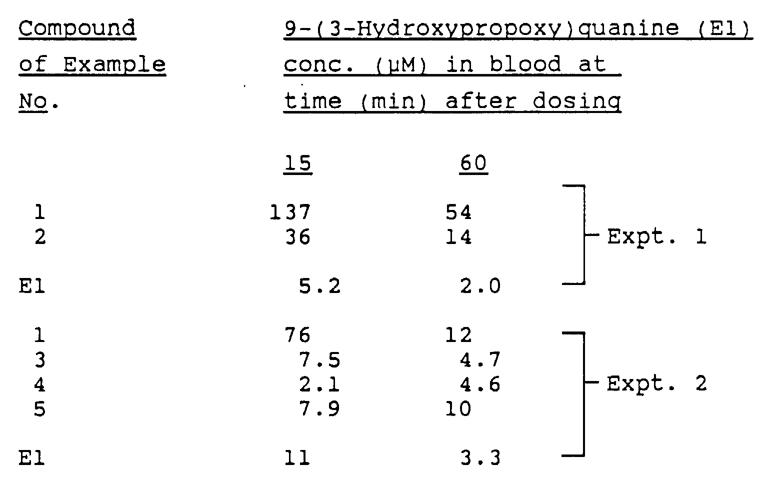

## Claims (Claims for the following Contracting State(s): AT, BE, CH, DE, DK, FR, GB, GR, IT, LI, LU, NL, SE)

1. A compound of formula (I) or a pharmaceutically acceptable salt thereof: 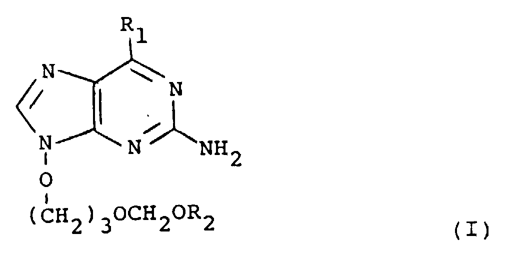 wherein
R₁ is hydrogen or hydroxy; and
R₂ is C₁₋₆ alkyl.

2. A compound according to claim 1 wherein R₁ is hydrogen.

3. A compound according to claim 1 or 2 wherein R₂ is iso-propyl.

4. A compound selected from the group consisting of:
2-amino-9-[3-(isopropoxymethoxy)propoxy)purine,
2-amino-9-[3-(methoxymethoxy)propoxy]purine,
9-[3-(isopropoxymethoxy)propoxy]guanine,
9-[3-(ethoxymethoxy)propoxy]guanine and
2-amino-9-[3-(ethoxymethoxy)propoxy]purine.

5. A process for the preparation of a compound according to claim 1, which process comprises either
i) imidazole ring closure of a compound of formula (II): 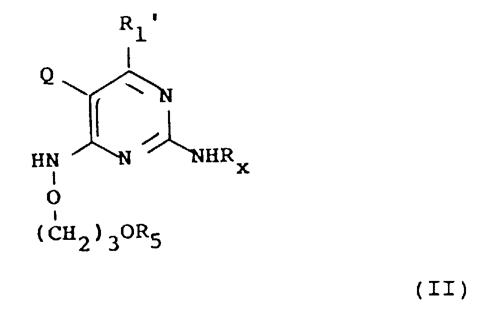 wherein Q is a group capable of cyclising to form an imidazole ring, such as amino or an amino derivative, for example, formylamino; or
ii) pyrimidine ring closure of a compound of formula (III): 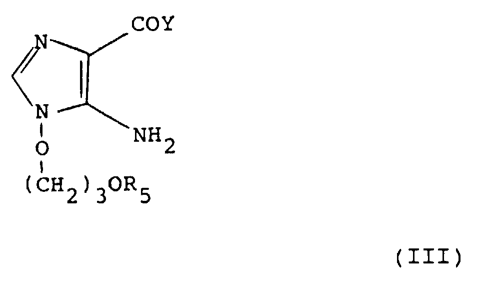 wherein Y is amino or C₁₋₆ alkoxy, with a condensing agent capable of cyclising to form a pyrimidine ring having a 2-NHRₓ substituent, resulting in a compound of formula (I) wherein R₁ is hydroxy; or
iii) condensing a compound of formula (IV): 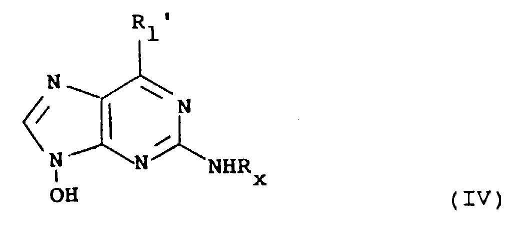 with a side chain intermediate of formula (V):
R₅O(CH₂)₃Z (V)
wherein Z is a leaving group;
and wherein, in formulae (II) to (V), R₅ is CH₂OR₂ or a group or atom convertible thereto, R₁′ is R₁ or a group or atom convertible thereto, Rₓ is hydrogen or an amino protecting group; and thereafter, when desired or necessary, converting R₅ and/or R₁′, when other than CH₂OR₂ and/or R₁, to CH₂OR₂ and/or R₁ respectively, and/or converting R₅ and/or R₁′ when CH₂OR₂ and/or R₁ to other CH₂OR₂ and/or R₁ and/or converting Rₓ when an amino protecting group, to hydrogen.

6. An intermediate of formula (II) as defined in claim 5, wherein R₅ is CH₂OR₂ wherein R₂ is as defined in claim 1.

7. An intermediate compound selected from:
4-[3-(isopropoxymethoxy)propoxyamine and
3-methoxymethoxypropoxyamine.

8. A pharmaceutical composition comprising a compound according to any one of claims 1 to 4, and a pharmaceutically acceptable carrier.

9. A compound according to any one of claims 1 to 4 for use as an active therapeutic substance.

10. A compound according to any one of claims 1 to 4 for use in treating viral infections.

11. Use of a compound according to any one of claims 1 to 4 in the manufacture of a medicament for use in the treatment of viral infections.

## Claims (Claims for the following Contracting State(s): ES)

1. A process for the preparation of a compound of formula (I) or a pharmaceutically acceptable salt thereof: 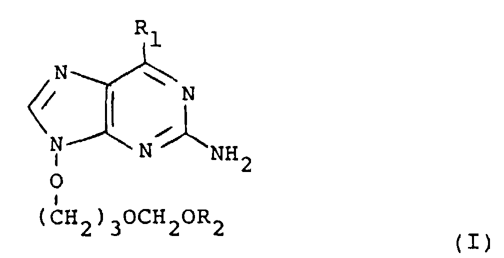 wherein
R₁ is hydrogen or hydroxy; and
R₂ is C₁₋₆ alkyl;
which process comprises either
i) imidazole ring closure of a compound of formula (II): 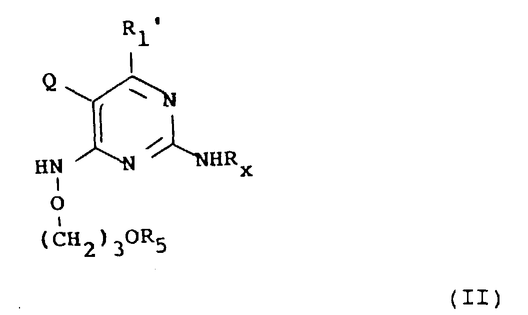 wherein Q is a group capable of cyclising to form an imidazole ring, such as amino or an amino derivative, for example, formylamino; or
ii) pyrimidine ring closure of a compound of formula (III): 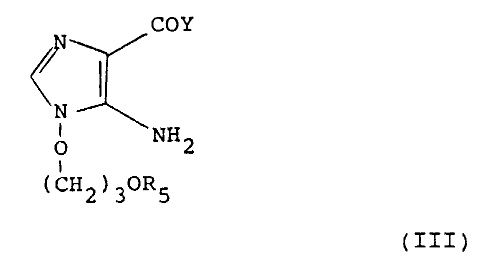 wherein Y is amino or C₁₋₆ alkoxy, with a condensing agent capable of cyclising to form a pyrimidine ring having a 2-NHRₓ substituent, resulting in a compound of formula (I) wherein R₁ is hydroxy; or
iii) condensing a compound of formula (IV): 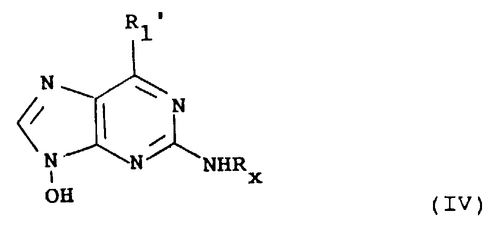 with a side chain intermediate of formula (V):
R₅O(CH₂)₃Z (V)
wherein Z is a leaving group;
and wherein, in formulae (II) to (V), R₅ is CH₂OR₂ or a group or atom convertible thereto, R₁′ is R₁ or a group or atom convertible thereto, Rₓ is hydrogen or an amino protecting group; and thereafter, when desired or necessary, converting R₅ and/or R₁′, when other than CH₂OR₂ and/or R₁, to CH₂OR₂ and/or R₁ respectively, and/or converting R₅ and/or R₁′ when CH₂OR₂ and/or R₁ to other CH₂OR₂ and/or R₁ and/or converting Rₓ when an amino protecting group, to hydrogen.

2. A process according to claim 1 wherein R₁ is hydrogen.

3. A process according to claim 1 or 2 wherein R₂ is iso-propyl.

4. A process according to claim 1 for the preparation of a compound selected from the group consisting of:
2-amino-9-[3-(isopropoxymethoxy)propoxy)purine,
2-amino-9-[3-(methoxymethoxy)propoxy]purine,
9-[3-(isopropoxymethoxy)propoxy]guanine,
9-[3-(ethoxymethoxy)propoxy]guanine and
2-amino-9-[3-(ethoxymethoxy)propoxy]purine.

5. An intermediate of formula (II) as defined in claim 1, wherein R₅ is CH₂OR₂ wherein R₂ is as defined in claim 1.

6. An intermediate compound selected from:
4-[3-(isopropoxymethoxy)propoxyamine and
3-methoxymethoxypropoxyamine.

7. Use of a compound of formula (I) as defined in claim 1,in the manufacture of a medicament for use in the treatment of viral infections.

## Patentansprüche (Patentansprüche für folgende(n) Vertragsstaat(en): AT, BE, CH, DE, DK, FR, GB, GR, IT, LI, LU, NL, SE)

1. Verbindung der Formel (I) oder ein pharmazeutisch verträgliches Salz davon: 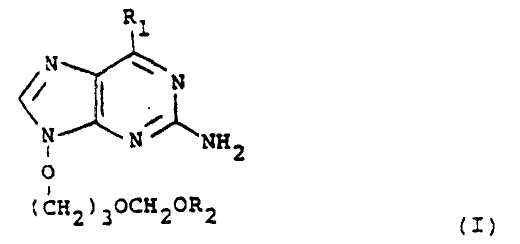 in der
R₁ ein Wasserstoffatom oder eine Hydroxylgruppe ist; und
R₂ ein C₁₋₆-Alkylrest ist.

2. Verbindung nach Anspruch 1, in der R₁ ein Wasserstoffatom ist.

3. Verbindung nach Anspruch 1 oder 2, in der R₂ eine iso-Propylgruppe ist.

4. Verbindung, ausgewählt aus der Gruppe:
2-Amino-9-[3-(isopropoxymethoxy)propoxy]purin,
2-Amino-9-[3-(methoxymethoxy)propoxy]purin,
9-[3-(Isopropoxymethoxy)propoxy]guanin,
9-[3-(Ethoxymethoxy)propoxy]guanin und
2-Amino-9-[3-(ethoxymethoxy)propoxy]purin.

5. Verfahren zur Herstellung einer Verbindung nach Anspruch 1. wobei das Verfahren entweder
i) einen Imidazolringschluß einer Verbindung der Formel (II): 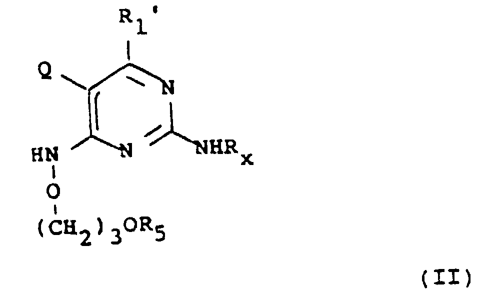 in der Q ein zur Zyklisierung unter Bildung eines Imidazolrings fähiger Rest, wie eine Aminogruppe oder ein Aminoderivat, zum Beispiel eine Formylaminogruppe, ist; oder
ii) einen Pyrimidinringschluß einer Verbindung der Formel (III): 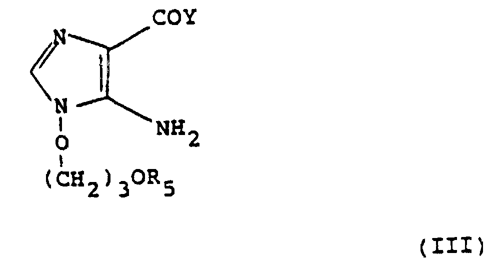 in der Y eine Aminogruppe oder ein C₁₋₆-Alkoxyrest ist, mit einem zur Zyklisierung unter Bildung eines Pyrimidinrings fähigen Kondensationsmittel mit einem 2-NHRₓ Substituenten, wobei eine Verbindung der Formel (I) erhalten wird, in der R₁ eine Hydroxylgruppe ist;
oder
iii) Kondensation einer Verbindung der Formel (IV): 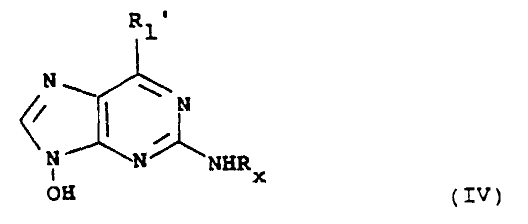 mit einem Seitenkettenzwischenprodukt der Formel (V):
R₅O(CH₂)₃Z (V)
in der Z eine Abgangsgruppe ist;
und wobei in den Formeln (II) bis (V) R₅ CH₂OR₂ oder ein dazu umwandelbarer Rest oder Atom ist, R₁' R₁ oder ein dazu umwandelbarer Rest oder Atom ist, Rₓ ein Wasserstoffatom oder eine Aminoschutzgruppe ist; und anschließend, falls gewünscht oder erforderlich, Umwandeln von R₅ und/oder R₁', wenn sie andere Reste als CH₂OR₂ und/oder R₁ sind, in jeweils CH₂OR₂ und/oder R₁, und/oder Umwandeln von R₅ und/oder R₁', wenn sie CH₂OR₂ und/oder R₁ sind, in andere Reste CH₂OR₂ und/oder R₁ und/oder Umwandeln von Rₓ, wenn es eine Aminoschutzgruppe ist, in ein Wasserstoffatom, umfaßt.

6. Zwischenprodukt der Formel (II) nach Anspruch 5, in dem R₅ CH₂OR₂ ist, wobei R₂ die in Anspruch 1 angegebene Bedeutung hat.

7. Zwischenprodukt, ausgewählt aus
4-[3-(Isopropoxymethoxy)propoxy]amin und
3-Methoxymethoxypropoxyamin.

8. Arzneimittel, umfassend eine Verbindung nach einem der Ansprüche 1 bis 4 und einen pharmazeutisch verträglichen Träger.

9. Verbindung nach einem der Ansprüche 1 bis 4 zur Verwendung als therapeutischen Wirkstoff.

10. Verbindung nach einem der Ansprüche 1 bis 4 zur Verwendung bei der Behandlung von Virusinfektionen.

11. Verwendung einer Verbindung nach einem der Ansprüche 1 bis 4 zur Herstellung eines Medikaments zur Verwendung bei der Behandlung von Virusinfektionen.

## Patentansprüche (Patentansprüche für folgende(n) Vertragsstaat(en): ES)

1. Verfahren zur Herstellung einer Verbindung der Formel (I) oder eines pharmazeutisch verträglichen Salzes davon: 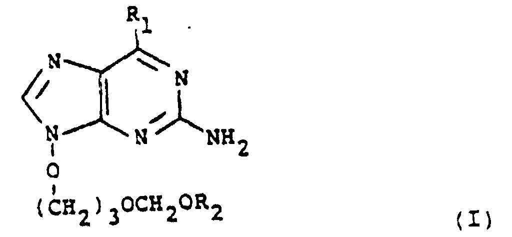 wobei
R₁ ein Wasserstoffatom oder eine Hydroxylgruppe ist; und
R₂ ein C₁₋₆-Alkylrest ist;
wobei das Verfahren entweder
i) einen Imidazolringschluß einer Verbindung der Formel (II): 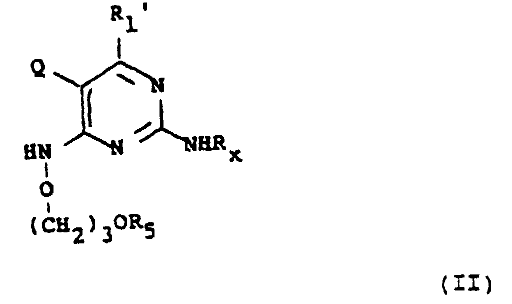 in der Q ein zur Zyklisierung unter Bildung eines Imidazolrings fähiger Rest, wie eine Aminogruppe oder ein Aminoderivat, zum Beispiel eine Formylaminogruppe, ist; oder
ii) einen Pyrimidinringschluß einer Verbindung der Formel (III): 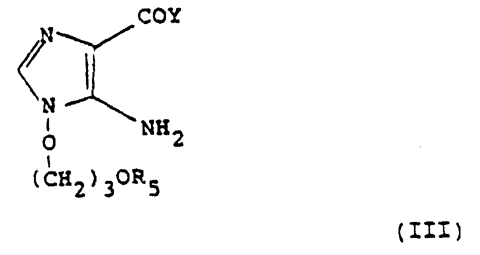 in der Y eine Aminogruppe oder ein C₁₋₆-Alkoxyrest ist, mit einem zur Zyklisierung unter Bildung eines Pyrimidinrings fähigen Kondensationmittel mit einem 2-NHRₓ Substituenten, wobei eine Verbindung der Formel (I) erhalten wird, in der R₁ eine Hydroxylgruppe ist;
oder
iii) Kondensation einer Verbindung der Formel (IV): 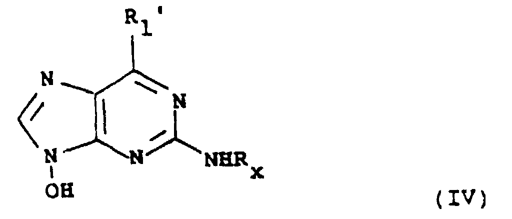 mit einem Seitenkettenzwischenprodukt der Formel (V):
R₅O(CH₂)₃Z (V)
in der Z eine Abgangsgruppe ist;
und wobei in den Formeln (II) bis (V) R₅ CH₂OR₂ oder ein dazu umwandelbarer Rest oder Atom ist, R₁' R₁ oder ein dazu umwandelbarer Rest oder Atom ist, Rₓ ein Wasserstoffatom oder eine Aminoschutzgruppe ist; und anschließend, falls gewünscht oder erforderlich, Umwandeln von R₅ und/oder R₁', wenn sie andere Reste als CH₂OR₂ und/oder R₁ sind, in jeweils CH₂OR₂ und/oder R₁, und/oder Umwandeln von R₅ und/oder R₁, wenn sie CH₂OR₂ und/oder R₁ sind, in andere Reste CH₂OR₂ und/oder R₁ und/oder Umwandeln von Rₓ, wenn es eine Aminoschutzgruppe ist, in ein Wasserstoffatom, umfaßt.

2. Verfahren nach Anspruch 1, wobei R₁ ein Wasserstoffatom ist.

3. Verfahren nach Anspruch 1 oder 2, wobei R₂ eine iso-Propylgruppe ist.

4. Verfahren nach Anspruch 1 zur Herstellung einer Verbindung, ausgewählt aus der Gruppe:
2-Amino-9-[3-(isopropoxymethoxy)propoxy]purin,
2-Amino-9-[3-(methoxymethoxy)propoxy]purin,
9-[3-(isopropoxymethoxy)propoxy]guanin,
9-[3-(Ethoxymethoxy)propoxy]guanin und
2-Amino-9-[3-(ethoxymethoxy)propoxy]purin.

5. Zwischenprodukt der Formel (II) nach Anspruch 1, in dem R₅ CH₂OR₂ ist, wobei R₂ die in Anspruch 1 angegebene Bedeutung hat.

6. Zwischenprodukt. ausgewählt aus
4-[3-(Isopropoxymethoxy)propoxy]amin und
3-Methoxymethoxypropoxyamin.

7. Verwendung einer Verbindung der Formel (I) nach Anspruch 1 zur Herstellung eines Arzneimittels zur Verwendung bei der Behandlung von Virusinfektionen.

## Revendications (Revendications pour l'(les) Etat(s) contractant(s) suivant(s): AT, BE, CH, DE, DK, FR, GB, GR, IT, LI, LU, NL, SE)

1. Composé de formule (I) ou un de ses sels pharmaceutiquement acceptables : 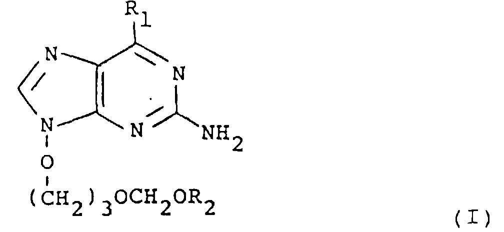 formule dans laquelle
R₁ représente l'hydrogène ou un groupe hydroxy ;
et R₂ représente un groupe alkyle en C₁ à C₆.

2. Composé suivant la revendication 1, dans lequel R₁ représente l'hydrogène.

3. Composé suivant la revendication 1 ou 2 , dans lequel R₂ représente un groupe isopropyle.

4. Composé choisi dans le groupe consistant en :
2-amino-9-[3-(isopropoxyméthoxy)propoxy)purine,
2-amino-9-[3-(méthoxyméthoxy)propoxy)purine,
9-[3-(isopropoxyméthoxy)propoxy)guanine,
9-[3-(éthoxyméthoxy)propoxy)guanine, et
2-amino-9-[3-(éthoxyméthoxy)propoxy)purine.

5. Procédé pour la préparation d'un composé suivant la revendication 1, procédé qui comprend
i) la cyclisation, formant un noyau imidazole, d'un composé de formule (II) : 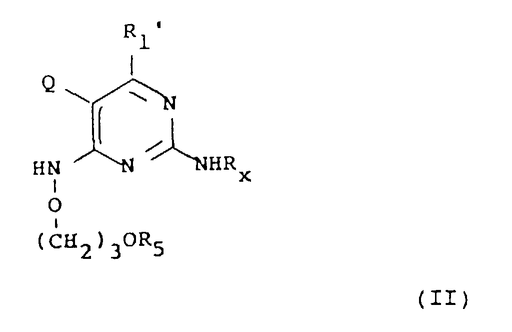 dans laquelle Q représente un groupe apte à la cyclisation pour former un noyau imidazole, tel qu'un groupe amino ou un dérivé à fonction amino, par exemple un groupe formylamino ; ou
ii) la cyclisation, formant un noyau pyrimidine, d'un composé de formule (III) : 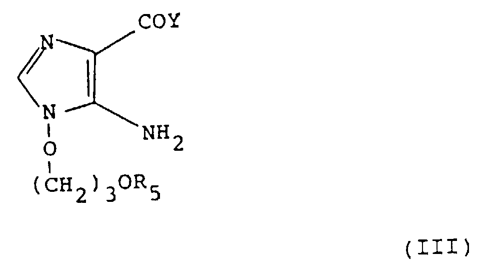 dans laquelle Y représente un groupe amino ou alkoxy en C₁ à C₆, avec un agent de condensation apte à la cyclisation pour former un noyau pyrimidine ayant un substituant 2-NHRₓ, avec pour résultat un composé de formule (I) dans laquelle R₁ représente un groupe hydroxy ;
ou
iii) la condensation d'un composé de formule (IV) : 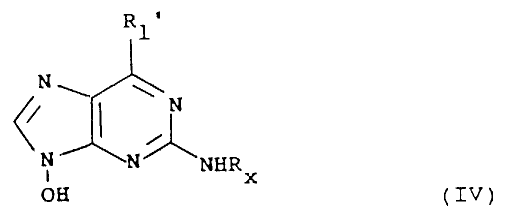 avec un intermédiaire de chaîne latérale de formule (V) :
R₅O(CH₂)₃Z (V)
dans laquelle Z représente un groupe partant ;
procédé dans lequel, dans les formules (II) à (V), R₅ représente un groupe CH₂OR₂ ou un groupe ou atome pouvant être transformé en ce groupe, R₁' représente un groupe R₁ ou un groupe ou atome pouvant être transformé en ce groupe, Rₓ représente l'hydrogène ou un groupe protecteur de la fonction amino ; puis, lorsque cela est désiré ou nécessaire, la transformation de R₅ et/ou R₁', lorsqu'ils représentent des groupes autres que CH₂OR₂ et/ou R₁, respectivement en des groupes CH₂OR₂ et/ou R₁, et/ou la transformation de R₅ et/ou R₁', lorsqu'ils représentent des groupes CH₂OR₂ et/ou R₁, en d'autres groupes CH₂OR₂ et/ou R₁, et/ou la transformation de Rₓ, lorsqu'il représente un groupe protecteur de la fonction amino, en l'hydrogène.

6. Intermédiaire de formule (II) répondant à la définition suivant la revendication 5, dans lequel R₅ représente un groupe CH₂OR₂ dans lequel R₂ répond à la définition suivant la revendication 1.

7. Composé intermédiaire choisi entre :
la 4-[3-(isopropoxyméthoxy)propoxyamine, et
la 3-méthoxyméthoxypropoxyamine.

8. Composition pharmaceutique comprenant un composé suivant l'une quelconque des revendications 1 à 4 et un support pharmaceutiquement acceptable.

9. Composé suivant l'une quelconque des revendications 1 à 4, destiné à être utilisé comme substance thérapeutique active.

10. Composé suivant l'une quelconque des revendications 1 à 4, destiné à être utilisé dans le traitement d'infections virales.

11. Utilisation d'un composé suivant l'une quelconque des revendications 1 à 4 dans la production d'un médicament destiné à être utilisé dans le traitement d'infections virales.

## Revendications (Revendications pour l'(les) Etat(s) contractant(s) suivant(s): ES)

1. Procédé pour la préparation d'un composé de formule (I) ou d'un de ses sels pharmaceutiquement acceptables : 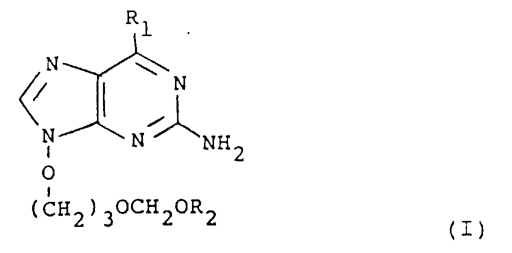 formule dans laquelle
R₁ représente l'hydrogène ou un groupe hydroxy ;
et R₂ représente un groupe alkyle en C₁ à C₆ ;
procédé qui comprend
i) la cyclisation, formant un noyau imidazole, d'un composé de formule (II) : 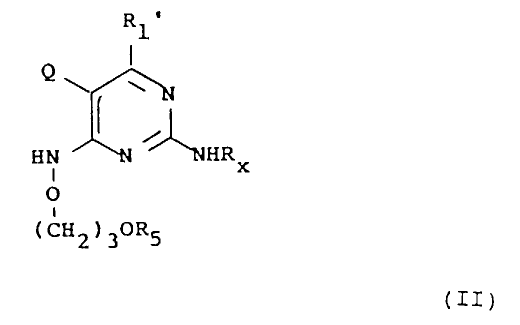 dans laquelle Q représente un groupe apte à la cyclisation pour former un noyau imidazole, tel qu'un groupe amino ou un dérivé à fonction amino, par exemple un groupe formylamino ; ou
ii) la cyclisation, formant un noyau pyrimidine, d'un composé de formule (III) : 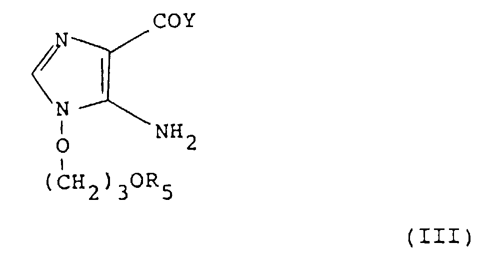 dans laquelle Y représente un groupe amino ou alkoxy en C₁ à C₆, avec un agent de condensation apte à la cyclisation pour former un noyau pyrimidine ayant un substituant 2-NHRₓ, avec pour résultat un composé de formule (I) dans laquelle R₁ représente un groupe hydroxy ;
ou
iii) la condensation d'un composé de formule (IV) : 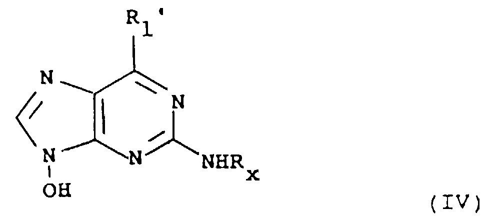 avec un intermédiaire de chaîne latérale de formule (V) :
R₅O(CH₂)₃Z (V)
dans laquelle Z représente un groupe partant ;
procédé dans lequel, dans les formules (II) à (V), R₅ représente un groupe CH₂OR₂ ou un groupe ou atome pouvant être transformé en ce groupe, R₁' représente un groupe R₁ ou un groupe ou atome pouvant être transformé en ce groupe, Rₓ représente l'hydrogène ou un groupe protecteur de la fonction amino ; puis, lorsque cela est désiré ou nécessaire, la transformation de R₅ et/ou R₁', lorsqu'ils représentent des groupes autres que CH₂OR₂ et/ou R₁, respectivement en des groupes CH₂OR₂ et/ou R₁, et/ou la transformation de R₅ et/ou R₁', lorsqu'ils représentent des groupes CH₂OR₂ et/ou R₁, en d'autres groupes CH₂OR₂ et/ou R₁, et/ou la transformation de Rₓ, lorsqu'il représente un groupe protecteur de la fonction amino, en l'hydrogène.

2. Procédé suivant la revendication 1, dans lequel R₁ représente l'hydrogène.

3. Procédé suivant la revendication 1 ou 2 , dans lequel R₂ représente un groupe isopropyle.

4. Procédé suivant la revendication 1 pour la préparation d'un composé choisi dans le groupe consistant en :
2-amino-9-[3-(isopropoxyméthoxy)propoxy)purine,
2-amino-9-[3-(méthoxyméthoxy)propoxy)purine,
9-[3-(isopropoxyméthoxy)propoxy)guanine,
9-[3-(éthoxyméthoxy)propoxy)guanine, et
2-amino-9-[3-(éthoxyméthoxy)propoxy)purine.

5. Intermédiaire de formule (II) répondant à la définition suivant la revendication 1, dans lequel R₅ représente un groupe CH₂OR₂ dans lequel R₂ répond à la définition suivant la revendication 1.

6. Composé intermédiaire choisi entre :
la 4-[3-(isopropoxyméthoxy)propoxyamine, et
la 3-méthoxyméthoxypropoxyamine.

7. Utilisation d'un composé de formule (I) répondant à la définition suivant la revendication 1 dans la production d'un médicament destiné à être utilisé dans le traitement d'infections virales.
